# DEMANDE DE BREVET EUROPEEN

(11) **EP 1 050 292 A2**
(43) Date de publication de la demande: **08.11.2000**
(21) Numéro de dépôt: 00400608.6
(22) Date de dépôt: 07.03.2000
(51) Int. Cl.: A61K 7/032

(54) **Utilisation d'un colorant polymérique sulfopolyester pour recourber les cils**

(30) Priorité: 28.04.1999 FR 9905384
(71) Demandeur: L'OREAL, 75008 Paris (FR)
(72) Inventeur: Collin, Nathalie, 92330 Sceaux (FR); De La Poterie, Valérie, 77820 Le Chatelet-en-Brie (FR)
(74) Mandataire: Kromer, Christophe

(57) **Abrégé**

L'invention a pour objet l'utilisation d'un colorant polymérique sulfopolyester dans une composition de revêtement des cils pour recourber les cils.

## Description

La présente invention a pour objet l'utilisation d'un colorant polymérique sulfopolyester dans une composition de revêtement des cils pour recourber les cils.

La composition peut être une composition de maquillage, une composition à appliquer sur un maquillage, dite encore top-coat, ou bien encore une composition de traitement des cils. Plus spécialement, la composition est un mascara.

Les compositions de revêtement des cils, appelées mascaras, comprennent généralement, de façon connue, au moins une cire, un polymère filmogène et une matière colorante pour déposer un film coloré de maquillage sur les cils et gainer ces derniers, comme le décrit par exemple le document WO-A-95/15741. Les matières colorantes utilisées sont généralement des pigments minéraux ou organiques ou des laques, ou bien encore des colorants solubles dans les milieux aqueux ou organiques. On utilise de préférence les oxydes minéraux car les laques organiques et les colorants solubles présentent un dégorgement trop important, tachant ainsi la peau et les lentilles oculaires.

Toutefois, ces compositions ne permettent pas d'obtenir un bon recourbement des cils.

Le but de la présente invention est de proposer une composition colorée de revêtement des cils conduisant, après application, à un revêtement conférant un bon recourbement des cils, tout en ne tachant pas la peau et les lentilles oculaires.

Les inventeurs ont découvert qu'un tel revêtement des cils pouvait être obtenu en utilisant un colorant polymérique sulfopolyester. Des compositions de mascara comprenant un tel colorant ont été décrites dans le document WO 98/26753. Ces compositions procurent un film coloré brillant.

Plus précisément, l'invention a pour objet l'utilisation d'un colorant polymérique sulfopolyester dans une composition de revêtement des cils pour recourber les cils.

L'invention a aussi pour objet un procédé pour recourber les cils, caractérisé par le fait qu'il comprend le revêtement des cils avec une composition cosmétique comprenant un polymère colorant sulfopolyester.

Après application de la composition contenant le colorant polymérique sulfopolyester, on constate que les cils sont bien recourbés et que le maquillage est brillant, ne tache pas la peau ou les lentilles oculaires. En outre, le maquillage présente une bonne tenue dans le temps, en particulier une bonne résistance aux frottements, notamment des doigts ou des tissus, ainsi qu'une bonne résistance à l'eau, à la transpiration et au sébum.

Par colorant polymérique, on entend un copolymère à base d'au moins deux monomères distincts dont l'un au moins est un colorant organique monomérique.

Les colorants polymériques sulfopolyesters peuvent résulter de la polymérisation de plusieurs monomères dont :
(i) au moins un résidu acide di-carboxylique portant au moins un groupement sulfonique;
(ii) au moins un résidu diol ; et
(iii) au moins un monomère colorant.

Les résidus acide dicarboxylique porteur de groupement sulfonique peuvent être choisis parmi les diacides cycloaliphatiques comme le sulfo diacide 1,4-cyclohexane carboxylique, ou bien encore parmi les diacides aromatiques tels que les acides sulfophtaliques, l'acide 4-sulfonaphtalène-2,7-dicarboxylique. Les résidus diols peuvent être choisi par exemple parmi l'éthylène glycol, le 1,2-propane diol, le 1,3-propane diol, le 2-méthyl-1,3-propanediol, le 1,4-butane diol, le 2,2-diméthyl-1,3-propanediol, le 1,6-hexanediol, le 1,10-décane diol, le 1,12-dodécane diol, le 1,2-cyclohexane diol, le 1,4-cyclohexanediol, le 1,2-cyclohexane diméthanol, le x,8-bis(hydroxyméthyl)-tricyclo-[5.2.1.0]décane, dans lequel x représente 3, 4 ou 5 ; les diols comprenant au moins un atome d'oxygène dans la chaîne comme par exemple le diéthylène glycol, le triéthylène glycol, le dipropylène glycol, le 1,3-bis (2-hydroxyéthyl) benzène, le 1,4-bis (2-hydroxyéthyl)benzène, etc.
En règle générale, ces diols comprennent 2 à 18 et préférentiellement 2 à 12 atomes de carbone.
En outre, les sulfopolyester colorant peuvent comporter des résidus acide di-carboxylique excempt de groupement sulfonique ; de tels résidus peuvent être de type aliphatique, alicyclique ou aromatique, comme par exemple les acides téréphtalique, isophtalique, 1,4-cyclohexane dicarboxylique, 1,3-cyclohexane di-carboxylique, succinique, glutarique, adipique, sébacique, 1, 2-dodécanedioïque, 2,6-naphtalène dicarboxylique, etc.

Les monomères colorants organiques selon l'invention doivent comporter au moins deux substituants susceptibles de réagir avec au moins l'un des autres monomères employés pour la préparation du colorant polymérique et doivent être stables à la température et dans les conditions de préparation dudit polymère.

Hormis ces deux conditions préliminaires, la nature chimique des monomères colorants n'a pas d'importance pour la réalisation de l'invention. Ceux-ci peuvent par exemple, être choisis parmi les anthraquinones, les méthines, bis-méthines, les aza-méthines, les arylidènes, les 3H-dibenzo[7,i-j] isoquinolines, les acides 2,5-diarylaminotéréphtaliques et leurs esters, les phtaloylphénothiazines, les phtaloylphénoxazines, les phtaloylacridones, les anthrapyrimidines, les anthrapyrazoles, les phtalocyanines, les quinophtalones, les indophénols, perinones, les nitroarylamines, benzodifurane, les 2 H-1-benzopyran-2-one, les quinophtalones, les perylènes, les quinacridones, les triphénodioxazines, les fluoridines, les 4-amino-1,8-naphtalimides, les thioxanthones, les benzanthrones, les indanthrones, les indigo, thioindigo, xanthène, acridine, azine, oxazine, etc.
On pourra se référer aux brevets US-4,267,306 ; US-4,359,570 ; US-4,403,092 ; US-4,617,373 ; US-4,080,355 ; US-4,740,581 ; US-4,116,923 ; US-4,745,173 ; US-4,804,719, US-5,194,463 ; W092/07913 pour trouver des exemples de monomères colorants utilisables dans la préparation des colorants polymériques.

Les substituants portés par le monomère colorant et susceptibles de réagir avec les autres monomères peuvent par exemple être choisis parmi les groupements suivants:
- hydroxy,
- carboxy, ester, amino, alkylamino : 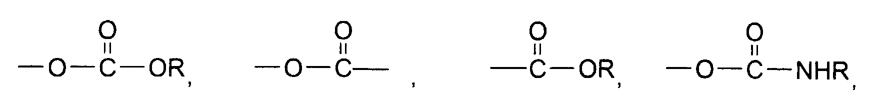 dans lesquels R représente un groupement choisi parmi les alkyles, les aryles. De préférence R est choisi parmi les groupements alkyles en C1-C8 et le phényle. Encore plus préférentiellement R est choisi parmi les groupements méthyle, éthyle et phényle.

Habituellement, le colorant polymérique comprend au moins 5 % en poids de monomère colorant, et n'en comprend pas plus de 55% en poids.
De préférence, le pourcentage en poids de monomère colorant par rapport au poids total du copolymère va de 10 à 40 %.

Pour la préparation des colorants polymériques, on peut se reporter aux procédés décrits dans le brevet US-5,804,719.

Selon un mode particulier de l'invention, on peut utiliser un polymère colorant dispersible dans l'eau ayant des groupes de liaison comprenant au moins environ 20 % en moles de carbonyloxy et jusqu'à environ 80 % en moles de carbonylamido, ledit polymère contenant des groupes sulfonate hydrosolubilisants et ayant d'environ 0,01 à environ 40 % en moles, sur la base du total de tous les équivalents d'hydroxy, de carboxy ou d'amino réactifs, de colorant comprenant un ou plusieurs composés organiques thermostables ayant initialement au moins un groupe condensable, que l'on a fait réagir sur ou dans le tronc polymère. Les équivalents susmentionnés englobent leurs divers dérivés condensables, y compris des carbalcoxy, carbaryloxy, N-alkylcarbamyloxy, acyloxy, chlorocarbonyle, carbamyloxy, N-(alkyl)₂ carbamyloxy, alkylamino, N-phénylcarbamyloxy, cyclohexanoyloxy et carbocyclohexyloxy.

Dans une forme préférée de la présente invention, le polymère colorant contient des groupes de liaison carbonyloxy dans la structure moléculaire linéaire, où jusqu'à 80 % desdits groupes de liaison peuvent être des groupes de liaison carbonylamido, le polymère ayant une viscosité inhérente d'environ 0,1 à environ 1,0, mesurée dans une solution à 60-40 parties en poids de phénol/tétrachloroéthane, à 25 °C et à une concentration de 0,25 gramme de polymère dans 100 ml du solvant, le polymère contenant des proportions essentiellement équimolaires d'équivalents d'acide (100 pour cent en moles) par rapport aux équivalents d'hydroxy et d'amino (100 pour cent en moles), le polymère comprenant les résidus de réaction des réactifs (a), (b), (c), (d) et (e) suivants ou de leurs dérivés générateurs d'ester ou générateurs d'estéramide :
(a) au moins un acide dicarboxylique difonctionnel ;
(b) d'environ 4 à environ 25 pour cent en moles, sur la base d'un total de tous les équivalents d'acide, d'hydroxyle et d'amino étant égal à 200 pour cent en moles, d'au moins 1 sulfomonomère difonctionnel contenant au moins un groupe sulfonate cationique relié à un noyau aromatique ou cycloaliphatique où les groupes fonctionnels sont des hydroxy, carboxyle ou amino ;
(c) au moins un réactif difonctionnel choisi parmi un glycol ou un mélange d'un glycol et d'une diamine ayant deux groupes -NRH, le glycol contenant deux groupes -CH₂-OH dont
   (1) au moins 10 pour cent en moles, sur la base du pourcentage total en moles d'équivalents d'hydroxy ou d'hydroxy et d'amino, sont un polyéthylèneglycol de formule structurale :

      H-(OCH₂-CH₂)ₙ-OH

      n étant un entier de 2 à environ 20 ou
   (2) d'environ 0,1 à moins d'environ 15 pour cent en moles, sur la base du pourcentage total en moles d'équivalents d'hydroxy ou d'hydroxy et d'amino, sont un polyéthylèneglycol de formule structurale :

      H-(OCH₂-CH₂)ₙ-OH

      n étant un entier compris entre 2 et environ 500, et à condition que le pourcentage en moles dudit polyéthylèneglycol dans ladite gamme soit inversement proportionnel à la valeur de n dans ladite gamme ;
(d) de zéro à au moins un réactif difonctionnel choisi parmi un acide hydroxycarboxylique ayant un groupe -C(R)₂-OH, un acide aminocarboxylique ayant un groupe -NRH, et un amino-alcool ayant un groupe - C(R)₂-OH et un groupe -NRH, ou des mélanges desdits réactifs difonctionnels ; où chaque R dans les réactifs (c) ou (d) est un atome H ou un groupe alkyle ayant de 1 à 4 atomes de carbone ; et
(e) d'environ 0,1 % en moles à environ 15 % en moles, sur la base d'un total de tous les équivalents d'acide, d'hydroxyle et d'amino étant égal à 200 % en moles, de colorant ayant au moins un groupe acide, hydroxy ou amino que l'on a fait réagir sur ou dans la chaîne polymère.

Avantageusement, le polymère colorant hydrodispersible comprend :
(a) un monomère acide comprenant de 75 mole % à 84 mole % d'acide isophtalique et de 25 mole % à 16 mole % de sel de sodium de l'acide sulfo-5-isophtalique,
(b) un monomère glycol comprenant de 45 à 60 mole % de diéthylène glycol et de 55 à 40 mole % de 1,4-cyclohexanediméthanol ou d'éthylèneglycol ou leur mélange,
(c) de 0,5 à 10 mole % de monomère colorant

De tels colorants polymériques sulfopolyesters sont décrits dans le brevet US-4,804,719.

Ces colorants polymériques sulfopolyesters peuvent être de type cristallin, semicristallin ou amorphe.

Habituellement, les colorants polymériques sulfopolyesters ont une viscosité intrinsèque d'au moins 0,20 (mesurée selon la méthode décrite dans le brevet US 4804719).

Le colorant polymérique utilisable dans la présente invention peut se présenter sous la forme d'une poudre dispersible ou soluble ou d'une solution ou d'une dispersion en milieu aqueux.

Une dispersion en milieu aqueux d'un colorant polymérique insoluble dans l'eau peut être préparée de façon connue à partir d'eau, de colorant polymérique brut tel que décrit dans les documents US-5,032,670 ; US-4,999,418 ; US-5,106,942 ; US-5,030,708; US-5,102,980 ; US-5,043,376, US-5,194,463. Par exemple on peut partir du colorant polymérique sulfopolyester sous forme de poudre ou de granulé, et d'au moins un tensio-actif ionique, de préférence un tensio-actif anionique ou amphotère. Les tensio-actifs anioniques et amphotères peuvent être choisis préférentiellement parmi les sels alcalins d'acides gras en C12-C24, les phosphatides de soja, les phospholipides, les lysophospholipides. Ces tensio-actifs ioniques sont introduits en quantités préférentiellement comprises entre 0,5 et 30%, et encore plus préférentiellement entre 1 et 10% en poids par rapport au poids du colorant polymérique sulfopolyester. De façon préférentielle, ces dispersions comprennent en outre au moins un tensioactif non-ionique, que l'on choisit avantageusement parmi les dérivés polyoxyéthylénés ayant un poids moléculaire supérieur à 300, préférablement aux alentours de 1000 à 15000 et une balance hydrophile-lipophile (ou balance HLB) supérieure ou égale à 10.

Par exemple, on peut préparer une dispersion aqueuse de colorant polymérique en suivant les étapes suivantes :
(i) préparation d'une émulsion huile dans eau à partir d'eau, d'une solution du colorant polymérique dans un solvant organique volatile dans lequel il est soluble, en présence d'au moins un tensio-actif ionique et éventuellement d'un tensio-actif non-ionique ;
(ii) évaporation du solvant organique volatil.

Le solvant organique volatile utilisable pour la mise en oeuvre de ce procédé doit être non miscible à l'eau et susceptible de solubiliser le polymère. De préférence son point d'ébullition est inférieur à 100°C. Il peut par exemple être choisi parmi : les solvants hydrocarbonés comme le n-hexane, le cyclohexane, le cyclopentane ; les solvants chlorés comme le chlorure de méthylène, le chloroforme ; les alkyl esters d'acides carboxyliques, comme l'acétate d'éthyle ; les dialkyléthers comme le diisopropyléther. On utilise préférentiellement pour la mise en oeuvre de ce procédé un mélange de solvants comprenant, outre les solvants décrits ci-dessus un solvant volatile, polaire, miscible à l'eau, comme l'acétone ou un alcanol de faible poids moléculaire.

Pour plus d'information sur un tel procédé, on peut se référer aux brevets US 5,043,376 et US 5,104,913.

L'eau des dispersions aqueuses décrites ci-dessus peut ensuite être évaporée, par exemple par atomisation ou par lyophilisation, afin d'obtenir une poudre d'une composition de colorant polymérique dispersible dans d'autres milieux.

Comme il vient d'être exposé, ce colorant polymérique peut être incorporé sous forme de solution ou de dispersion aqueuse ou tel quel, sous forme de poudre dispersible ou de poudre brute. De préférence, le colorant polymérique polyester peut être incorporé sous forme de dispersion aqueuse ou sous forme de poudre dispersible dans l'eau.

Les colorants polymériques utilisables dans la composition pour l'utilisation selon l'invention peuvent être introduits en quantités allant de 0,1 % à 60% en poids de matières sèches, par rapport au poids total de la composition, de préférence de 0,1 % à 40 % en poids et mieux de 0,5 % à 30 % en poids.

Les colorants polymériques décrits ci-dessus ont l'avantage de présenter la même structure chimique générale et les mêmes propriétés physico-chimiques, dans des gammes de couleurs très différentes. Ainsi, un pigment rouge aura le même comportement qu'un pigment de couleur jaune. Cette homogénéité de comportement permet la fabrication des compositions de maquillages sous forme de monochromes. Lors du mélange des monochromes pour la réalisation d'une teinte, on n'observe aucun problème d'incompatibilité entre les différentes couleurs.

La cire présente dans la composition pour l'utilisation selon l'invention peut être choisie parmi les cires d'origine animale, les cires d'origine végétale, les cires d'origine minérale, les cires synthétiques et les fractions diverses de cires d'origine naturelle. Les cires peuvent être présentes en une teneur allant de 2 % à 40 % en poids, par rapport au poids total de la composition, de préférence de 5 % à 30 % en poids, et mieux de 10 % à 25 % en poids.

Avantageusement, la cire peut être choisie parmi les cires (I) ayant un point de fusion allant de 70 °C à 110 °C. Ces cires ont notamment une pénétrabilité à l'aiguille allant de 1 à 7, 5. La pénétrabilité à l'aiguille des cires est déterminée selon la norme française NF T 60-123 ou la norme américaine ASTM D 1321, à la température de 25 °C. Selon ces normes, la pénétrabilité à l'aiguille est la mesure de la profondeur, exprimée en dixièmes de millimètre, à laquelle une aiguille normalisée, pesant 2,5 g disposée dans un équipage mobile pesant 97,5 g et placée sur la cire à tester, pendant 5 secondes, pénètre dans la cire.

Les cires (I) peuvent par exemple être choisies notamment parmi la cire de son de riz, la cire de Carnauba, la cire d'Ouricuri, la cire de Candellila, les cires de Montan, la cire de canne à sucre, les ozokérites, certaines cires de polyéthylène qui répondent aux critères des cires (I).

Avantageusement, la composition pour l'utilisation selon l'invention peut comprendre une quantité de cires (I) allant de 0,1 % à 20 % en poids, par rapport au poids total de la composition, de préférence de 1 % à 10 % en poids.

Selon un mode de réalisation selon l'invention, la composition peut comprendre au moins une cire (la) ayant un point de fusion supérieur ou égal à 70 °C et inférieur à 83 °C et/ou une cire (lb) ayant un point de fusion allant de 83 °C à 110 °C.

Comme cire (la), on peut par exemple citer la cire de son de riz ou la cire de Candelilla. Comme cire (lb), on peut citer par exemple la cire de Carnauba, la cire d'Ouricuri, les cires de Montan. On utilise de préférence la cire de Carnauba.

Avantageusement, la composition pour l'utilisation selon l'invention peut comprendre un mélange de cires (I) contenant au moins une première cire (la) et au moins une deuxième cire (lb) telles que définies précédemment.

Ledit mélange de cires (I) peut comprendre de 5 % à 50 % en poids de cire (la), par rapport au poids total dudit mélange de cires (I), et de 50 % à 95 % en poids de cire (lb).

La composition peut comprendre, en outre, au moins une cire (II), dite cire molle, ayant un point de fusion supérieur ou égal à 45 °C et inférieur à 70 °C. La cire (II) peut avantageusement avoir une pénétrabilité à l'aiguille supérieure à 7,5, et de préférence inférieure ou égale à 217, mesurée selon les conditions définies précédemment pour les cires (I). Cette cire (II) permet notamment d'assouplir le revêtement déposé sur les cils.

Ces cires (II) peuvent être notamment choisies parmi la cire d'abeilles, les cires de lanoline, les cires de paraffine, les cires de cérasine, les cires microcristallines, les spermaceti, certaines cires de polyéthylène de poids moléculaire tel qu'elles répondent aux critères des cires (II), les huiles végétales hydrogénées.

Parmi les huiles végétales hydrogénées, on peut citer les cires de jojoba hydrogénées et les huiles hydrogénées qui sont obtenues par hydrogénation catalytique de corps gras composés de chaîne grasse linéaire ou non en C₈-C₃₂ et qui ont les qualités correspondant à la définition des cires. On peut citer notamment l'huile de tournesol hydrogénée, l'huile de ricin hydrogénée, l'huile de coton hydrogénée, l'huile de coprah hydrogénée et la lanoline hydrogénée.

Avantageusement, la cire (I) et la cire (II) peuvent être présentes dans la composition selon un rapport pondéral cire (I) / cire (II) pouvant aller de 0,2 à 1, et de préférence de 0,4 à 0,7.

La composition pour l'utilisation selon l'invention peut comprendre de l'eau et se présenter sous la forme de dispersion cire-dans-eau, eau-dans cire, huile-dans-eau et eau-dans-huile. La teneur en eau dans la composition peut aller de 1 à 95 % en poids, par rapport au poids total de la composition, et mieux de 10 à 80 % en poids.

La composition pour l'utilisation selon l'invention peut comprendre en outre au moins une huile volatile. On entend par "huile volatile" une huile susceptible de s'évaporer à température ambiante d'un support sur lequel elle a été appliquée, autrement dit une huile ayant une tension de vapeur mesurable à température ambiante.

On peut notamment utiliser une ou plusieurs huiles volatiles à température ambiante et pression atmosphérique ayant par exemple une tension de vapeur, à pression et température ambiante > 0 mm de Hg (0 Pa) et en particulier allant de 10⁻³ à 300 mm de Hg (0,13 Pa à 40.000 Pa), à condition que la température d'ébullition soit supérieure à 30 °C. Ces huiles volatiles sont favorables à l'obtention d'un film à propriétés "sans transfert" total et de bonne tenue. Ces huiles volatiles facilitent, en outre, l'application de la composition sur la peau, les muqueuses, les phanères. Ces huiles peuvent être des huiles hydrocarbonées, des huiles siliconées, des huiles fluorées, ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles préférées convenant pour la composition selon l'invention sont en particulier les huiles hydrocarbonées ayant de 8 à 16 atomes de carbones, et notamment les isoalcanes en C8-C16 (ou isoparaffines) et les esters ramifiés en C8-C16 comme l'isododécane (encore appelé 2,2,4,4,6-pentaméthylheptane), l'isodécane, l'isohexadécane, le néo pentanoate d'iso-hexyle, et leurs mélanges. D'autres huiles hydrocarbonées volatiles comme les distillats de pétrole, notamment ceux vendus sous la dénomination Shell Solt par la société SHELL, peuvent aussi être utilisées.

Comme huiles volatiles, on peut aussi utiliser les silicones volatiles, comme par exemple les huiles de silicones cycliques et volatiles, notamment celles ayant une viscosité ≤8 centistokes (8 10⁻⁶ m²/s), telles que l'octaméthylcyclotétrasiloxane, le décaméthylcyclopentasiloxane, le dodécaméthylcyclohexasiloxane, les silicones linéaires volatiles telles que l'octaméthyltrisiloxane, l'heptaméthylhexyltrisiloxane, l'heptaméthyloctyltrisiloxane, le décaméthyltétrasiloxane, ou bien encore les huiles volatiles fluorées telles que le nonafluorométhoxybutane ou le perfluorométhylcyclopentane.

L'huile volatile peut être présente dans la composition pour l'utilisation selon l'invention en une teneur allant de 0 % à 80 % en poids (notamment de 1 % à 80 %), par rapport au poids total de la composition, de préférence de 0 % à 65 % en poids (notamment de 1 % à 65 %).

La composition peut également comprendre au moins une huile non volatile, et notamment choisie parmi les huiles hydrocarbonées et/ou siliconées et/ou fluorées non volatiles.
Comme huile hydrocarbonée non volatile, on peut notamment citer :
- les huiles hydrocarbonées d'origine animale telle que le perhydrosqualène ;
- les huiles hydrocarbonées d'origine végétale telles que les triglycérides liquides d'acides gras de 4 à 10 atomes de carbone comme les triglycérides des acides heptanoïques ou octanoïque, ou encore les huiles de tournesol, de pépins de raisin, de sésame, de maïs, d'abricot, de ricin, d'avocat, d'olive ou de germes de céréales de soja, l'huile d'amande douce, de palme, de colza, de coton, de noisette, de macadamia, de jojoba, les triglycérides des acides caprylique/caprique comme ceux vendus par la société Stéarineries Dubois ou ceux vendus sous les dénominations Miglyol 810, 812 et 818 par la société Dynamit Nobel, l'huile de beurre de karité ;
- les hydrocarbures linéaires ou ramifiés, d'origine minérale ou synthétique tels que la vaseline, les polydécènes, le polyisobutène hydrogéné tel que le parléam ;
- les esters et les éthers de synthèse comme les huiles de formule R₁COOR₂ dans laquelle R₁ représente le reste d'un acide gras supérieur comportant de 6 à 29 atomes de carbone et R₂ représente une chaîne hydrocarbonée contenant de 3 à 30 atomes de carbone, telles que l'huile de Purcellin, le myristate d'isopropyle, le palmitate d'isopropyle, le stéarate de butyle, le laurate d'hexyle, l'adipate de diisopropyle, l'isononate d'isononyle, le palmitate de 2-éthyl-hexyle, le laurate de 2-hexyl-décyle, le palmitate de 2-octyl-décyle, le myristate ou le lactate de 2-octyl-dodécyle ; les esters de polyols comme le dioctanoate de propylène glycol, le diheptanoate de néopentylglycol, le diisononanoate de diéthylène glycol et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide myristique, l'acide palmitique, l'acide stéarique, l'acide béhénique, l'acide oléique, l'acide linoléique, l'acide linolénique ou l'acide isostéarique ;
et leurs mélanges.

Les huiles de silicone non volatiles utilisables dans la composition selon l'invention peuvent être des huiles de faible viscosité telles que les polysiloxanes linéaires dont le degré de polymérisation est de préférence de 6 à 2000 environ. On peut citer, par exemple, les polydiméthylsiloxanes (PDMS) de viscosité supérieure à 10 mPa.s, les phényl diméthicones, les phényl triméthicones, les polyphénylméthylsiloxanes et leurs mélanges.

Les huiles fluorées utilisables dans l'invention sont notamment des huiles fluorosiliconées, des polyéthers fluorés, des silicones fluorées telles que décrit dans le document EP-A-847752.

Les huiles non volatiles peuvent être présentes dans la composition selon l'invention en une teneur allant de 0 % à 50 % en poids (notamment 0,1 à 50 %), par rapport au poids total de la composition, de préférence de 0 % à 20 % en poids (notamment 0,1 % à 20 %).

La composition peut comprendre un polymère filmogène additionnel différent du colorant polymérique sulfopolyester défini précédemment. En particulier, le polymère filmogène additionnel peut être solubilisé ou dispersé dans le milieu de la composition, notamment dans un milieu aqueux.

Le polymère filmogène additionnel peut être choisi parmi :
- les dérivés de kératine, tels que les hydrolysats de kératine et les kératines sulfoniques ;
- les dérivés de chitine ou de chitosane anioniques, cationiques, amphotères ou non-ioniques ;
- les dérivés de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères ou copolymères acryliques, tels que les polyacrylates ou les polyméthacrylates ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; l'alcool polyvinylique ;
- les polyesters, notamment les polymères polyester et/ou polyesteramide anioniques dispersibles dans l'eau, comprenant des monomères portant une fonction :-SO₃M, avec M représentant un atome d'hydrogène, un ion ammonium NH₄⁺ ou un ion métallique, comme par exemple un ion Na⁺, Li⁺, K⁺, Mg²⁺, Ca²⁺, Cu²⁺, Fe²⁺, Fe³⁺. On peut citer en particulier les polymères décrits dans les documents US-3,734,874 ; US-4,233,196 ; US-4,304,901. Avantageusement, on choisit des polymères polyesters filmogènes à base d'au moins un acide dicarboxylique, d'au moins un diol et d'au moins un monomère aromatique bifonctionnel portant en outre un groupement -SO₃M tel que décrit ci-dessus ;
- les polyesters à chaîne grasse, les polyamides, et les résines époxyesters ;
- les polymères polyuréthannes, notamment les polyuréthannes anioniques, cationiques, non ioniques ou amphotères, les polyuréthanne-acryliques, les polyuréthanne-polyvinylpyrrolidones, les polyester-polyuréthannes, les polyéther-polyuréthannes, les polyurées, les polyurée/polyuréthannes,
- les polymères d'origine naturelle, éventuellement modifiés, tels que :
   . les gommes arabiques, la gomme de guar, les dérivés du xanthane, la gomme de karaya ;
   . les alginates et les carraghénates ;
   . les glycoaminoglycanes, l'acide hyaluronique et ses dérivés ;
   . la résine shellac, la gomme de sandaraque, les dammars, les élémis, les copals ;
   . l'acide désoxyribonucléïque ;
et leurs mélanges.

Le polymère filmogène additionnel peut être présent dans la composition en une teneur en matière sèche allant de 0,1 % à 10 % en poids, par rapport au poids total de la composition.

La composition pour l'utilisation selon l'invention peut contenir des agents tensioactifs émulsionnants présents notamment en une proportion allant de 2 à 30 % en poids par rapport au poids total de la composition, et mieux de 5 % à 15 %. Ces agents tensioactifs peuvent être choisis parmi des agents tensioactifs anioniques ou non ioniques. On peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs utilisés préférentiellement dans la composition sont choisis :
- parmi les tensioactifs non-ioniques : les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de C₁-C₆ alkyl glucose polyoxyéthylénés.
- parmi les tensioactifs anioniques : les acides gras en C16-C30 neutralisés par les amines, l'ammoniaque ou les sels alcalins.

On utilise de préférence des tensioactifs permettant l'obtention d'émulsion huile-dans-eau ou cire-dans-eau.

La composition peut également comprendre au moins une matière colorante comme les composés pulvérulents, par exemple à raison de 0,01 à 25 % du poids total de la composition. Les composés pulvérulents peuvent être choisis parmi les pigments et/ou les nacres et/ou les charges habituellement utilisés dans les mascaras.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges peuvent être choisies parmi celles bien connues de l'homme du métier et couramment utilisées dans les compositions cosmétiques.

La composition pour l'utilisation selon l'invention peut également contenir des ingrédients couramment utilisés en cosmétique, tels que les oligo-éléments, les adoucissants, les séquestrants, les parfums, les huiles, les silicones, les épaississants, les vitamines, les protéines, les céramides, les plastifiants, les agents de cohésion ainsi que les agents alcalinisants ou acidifiants habituellement utilisés dans le domaine cosmétique, les émollients, les conservateurs.

Bien entendu, l'homme du métier veillera à choisir ce ou ces éventuels composés complémentaires, et/ou leurs quantités, de manière telles que les propriétés avantageuses de la composition selon l'invention ne soient pas, ou substantiellement pas, altérées par l'adjonction envisagée.

La composition pour l'utilisation selon l'invention peut être préparée selon les méthodes usuelles des domaines considérés.

L'invention est illustrée plus en détail dans les exemples suivants.

### Exemple 1 :

On a préparé une composition de mascara ayant la composition suivante :
- Colorant sulfopolyester jaune (décrit à l'exemple 44 dans EP-A-747036) 11,25 g MA
- Colorant sulfopolyester rouge (décrit à l'exemple 10 dans EP-A-747036) 4,5 g MA
- Colorant sulfopolyester bleu (décrit à l'exemple 13 dans EP-A-747036) 7,5 g MA
- Agent épaississant (Serad FX 1100 de la société HULS) 0,6 g MA
- Agent d'étalement 0,9 g
- Eau qs 100 g

Après application du mascara sur les cils, on obtient un bon recourbement des cils et un maquillage noir très brillant, présentant une bonne tenue (résistant aux frottements). Le mascara ne tache pas la peau.

### Exemple 2 :

On a préparé une composition de mascara ayant la composition suivante :
- Cire de carnauba 5,55 g
- Cire de son de riz 5,66 g
- Cire d'abeille 4,8 g
- Cire de candelilla 1,9 g
- Polymères filmogènes hydrosolubles 0,4 g
- Gomme arabique 1,15 g
- Acide stéarique 4,1 g
- Triéthanolamine 1,8 g
- Colorant sulfopolyester jaune (décrit à l'exemple 44 dans EP-A-747036) 3,63 g MA
- Colorant sulfopolyester rouge (décrit à l'exemple 10 dans EP-A-747036) 1,45 g MA
- Colorant sulfopolyester bleu (décrit à l'exemple 13 dans EP-A-747036) 2,4 g MA
- Eau qs 100 g

On obtient un mascara noir qui confère un bon recourbement des cils et présente une bonne tenue (résistant aux frottements). Le mascara ne tache pas la peau.

## Revendications

1. Utilisation d'un colorant polymérique sulfopolyester dans une composition de revêtement des cils pour recourber les cils.

2. Utilisation selon la revendication 1, caractérisée par le fait que le colorant polymérique sulfopolyester résulte de la polymérisation de plusieurs monomères dont :
(i) au moins un résidu acide di-carboxylique portant au moins un groupement sulfonique;
(ii) au moins un résidu diol ; et
(iii) au moins un monomère colorant.

3. Utilisation selon l'une des revendications 1 ou 2, caractérisée en ce que le colorant polymérique sulfopolyester est un polymère colorant dispersible dans l'eau ayant des groupes de liaison comprenant au moins environ 20 % en moles de carbonyloxy et jusqu'à environ 80 % en moles de carbonylamido, ledit polymère contenant des groupes sulfonate hydrosolubilisants et ayant d'environ 0,01 à environ 40 % en moles, sur la base du total de tous les équivalents d'hydroxy, de carboxy ou d'amino réactifs, de colorant comprenant un ou plusieurs composés organiques thermostables ayant initialement au moins un groupe condensable, que l'on a fait réagir sur ou dans le tronc polymère

4. Utilisation selon l'une quelconque des revendications précédentes, caractérisée en ce que le colorant polymérique comprend 5 % à 55% en poids d'au moins un monomère colorant.

5. Utilisation selon la revendication 4, caractérisée en ce que le colorant polymérique comprend 10% à 40% en poids d'au moins un monomère colorant.

6. Utilisation selon l'une quelconque des revendications précédentes, caractérisée en ce que le colorant polymérique sulfopolyester est un polymère colorant hydrodispersible comprenant :
(a) un monomère acide comprenant de 75 mole % à 84 mole % d'acide isophtalique et de 25 mole % à 16 mole % de sel de sodium de l'acide sulfo-5-isophtalique,
(b) un monomère glycol comprenant de 45 à 60 mole % de diéthylène glycol et de 55 à 40 mole % de 1,4-cyclohexanediméthanol ou d'éthylèneglycol ou leur mélange,
(c) de 0,5 à 10 mole % de monomère colorant.

7. Utilisation selon l'une quelconque des revendications 2 à 6, caractérisée en ce que le monomère colorant est choisi dans le groupe formé par les anthraquinones, les méthines, bis-méthines, les aza-méthines, les arylidènes, les 3H-dibenzo[7,i-j] isoquinolines, les acides 2,5-diarylaminotéréphtaliques et leurs esters, les phtaloylphénothiazines, les phtaloylphénoxazines, les phtaloylacridone, les anthrapyrimidines, les anthrapyrazoles, les phtalocyanines, les quinophtalones, les indophénols, perinones, les nitroarylamines, benzodifurane, les 2 H-1-benzopyran-2-one, les quinophtalones, les perylènes, les quinacridones, les triphénodioxazines, les fluoridines, les 4-amino-1,8-naphtalimides, les thioxanthones, les benzanthrones, les indanthrones, les indigo, thioindigo, xanthène, acridine, azine, oxazine.

8. Utilisation selon l'une quelconque des revendications précédentes, caractérisée en ce que la composition comporte de 0,1 % à 60% en poids de matières sèches de colorant polymérique, de préférence de 0,1 % à 40 % en poids et mieux de 0,5 % à 30 % en poids.

9. Utilisation selon l'une quelconque des revendications précédentes, caractérisée par le fait que la composition comprend en outre au moins une cire.

10. Utilisation selon la revendication 9, caractérisée par le fait que la cire est présente en une teneur allant de 2 % à 40 % en poids, par rapport au poids total de la composition, de préférence de 5 % à 30 % en poids, et mieux de 10 % à 25 % en poids.

11. Utilisation selon l'une quelconque des revendications précédentes, caractérisée par le fait que la composition comprend au moins une cire (I) ayant un point de fusion allant de 70 °C à 110 °C.

12. Utilisation selon la revendication 11, caractérisée par le fait que la cire (I) est présente en une teneur allant de 0,1 % à 20 % en poids, par rapport au poids total de la composition.

13. Utilisation selon l'une quelconque des revendications précédentes, caractérisée par le fait que la composition comprend au moins une cire (II) ayant un point de fusion supérieur ou égal à 45 °C et inférieur à 70 °C.

14. Utilisation selon la revendication 13, caractérisée par le fait que la cire (II) est présente selon un rapport pondéral cire (I)/cire (II) allant de 0,2 à 1.

15. Utilisation selon l'une quelconque des revendications précédentes, caractérisée par le fait que la composition comprend un polymère filmogène additionnel différent du colorant polymérique sulfopolyester.

16. Utilisation selon l'une quelconque des revendications précédentes, caractérisée par le fait que la composition comprend au moins un additif choisi dans le groupe formé par les pigments, les nacres, les charges, les oligo-éléments, les adoucissants, les séquestrants, les parfums, les huiles, les silicones, les épaississants, les vitamines, les protéines, les céramides, les plastifiants, les agents de cohésion, les agents alcalinisants, les agents acidifiants, les émollients, les conservateurs.

17. Procédé pour recourber les cils, caractérisé par le fait qu'il comprend le revêtement des cils avec une composition cosmétique comprenant un colorant polymérique sulfopolyester tel que défini selon l'une quelconque des revendications 1 à 7.

18. Procédé selon la revendication 17, caractérisé par le fait que la composition est une composition selon l'une quelconque des revendications 8 à 16.
